(19) Europäisches Patentamt
European Patent Office
Office européen des brevets

(11) EP 2 708 879 A1

(12) **EUROPÄISCHE PATENTANMELDUNG**

(43) Veröffentlichungstag:
**19.03.2014 Patentblatt 2014/12**

(51) Int Cl.:
***G01N 27/22*** *(2006.01)*

(21) Anmeldenummer: **12184464.1**

(22) Anmeldetag: **14.09.2012**

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**
Benannte Erstreckungsstaaten:
**BA ME**

(71) Anmelder: **Baumer Electric AG
8501 Frauenfeld (CH)**

(72) Erfinder:
• **Brändle, Daniel
8500 Frauenfeld (CH)**
• **Hafner, Ueli
8413 Neftenbach (CH)**

(74) Vertreter: **Strauss, Steffen
Baumer Innotec AG
Hummelstrasse 17
Group Intellectual Property
8501 Frauenfeld (CH)**

(54) **Messanordnung zum Bestimmen einer Messkapazität einer Messprobe**

(57)     Die Erfindung betrifft eine Messanordnung zum Bestimmen einer Messkapazität einer Messprobe, umfassend einen Schwingkreis (100) umfassend einen Kapazitätssensor (101) und eine erste Induktivität (105), welche mit dem Kapazitätssensor (101) elektrisch verbunden ist, und einen Anregungskreis (200) umfassend eine zweite Induktivität (205), welche mit der ersten Induktivität (105) magnetisch koppelbar ist, um ein Anregungssignal zum Überführen des Schwingkreises (100) in einen Resonanzbetrieb zu übertragen, einen Anregungssignalgenerator (203), welcher mit der zweiten Induktivität (205) elektrisch verbunden und ausgebildet ist, das Anregungssignal zu erzeugen, und eine Auswerteeinrichtung (201), welche ausgebildet ist, die Messkapazität aus einer in dem Resonanzbetrieb erfassbaren elektrischen Größe zu bestimmen.

Fig. 1

EP 2 708 879 A1

**Beschreibung**

Die vorliegende Erfindung betrifft das Gebiet der Kapazitätsmessung.

Zur Erfassung einer physikalischen Größe wie beispielsweise einer Messkapazität eines Messobjektes werden üblicherweise Messsensoren eingesetzt, deren Messsignale üblicherweise zur Auswerteeinrichtungen übertragen werden müssen.

Die Übertragung der Messsignale kann beispielsweise über eine galvanische Kabelverbindung erfolgen. Dies ist jedoch in Umgebungen, in welchen kein Kabelanschluss möglich ist oder eine elektrische Kontaktierung nicht zuverlässig realisiert werden kann, nicht realisierbar. Derartige Umgebungen sind beispielsweise Bereiche mit hohen Umweltbelastungen. Befindet sich ein Messobjekt, wie beispielsweise Blut, in einer Zentrifuge, so ist eine galvanische Übertragung der Messsignale zu einer stationären Auswerteeinrichtung problematisch.

Ferner sind passive Mikrowellen-Resonanzsensoren zur kontaktlosen Übertragung von Sensorerfassungssignalen bekannt. Jedoch ist eine Aufbereitung der Sensorerfassungssignale in Form von Verstärkung schwacher Empfangssignale aufwändig.

Es ist die Aufgabe der vorliegenden Erfindung, ein effizientes Konzept zur kontaktlosen Erfassung von Sensorsignalen bereitzustellen.

Diese Aufgabe wird durch Gegenstände mit den Merkmalen nach den unabhängigen Ansprüchen gelöst. Vorteilhafte Ausführungsformen der Erfindung sind Gegenstand der abhängigen Ansprüche, der Beschreibung und der Figuren.

Die Erfindung basiert auf der Erkenntnis, dass diese Aufgabe durch eine Messanordnung gelöst werden kann, welche zwei Messteile umfasst, welche miteinander magnetisch koppelbar sind, nämlich einen Schwingreis mit einem Kapazitätssensor sowie einer Sekundärspule und einem Anregungskreis mit einer Primärspule sowie einer Auswerteeinrichtung. Die Primärspule und die Sekundärspule bilden einen Transformator, über welchen der Anregungskreis und der Schwingkreis magnetisch koppelbar sind.

Der Schwingkreis kann daher in einem kritischen Bereich mit hohen Umweltbelastungen, wo die Messung stattfindet, angeordnet sein, während der Anregungskreis in einem unkritischen Bereich angeordnet werden kann, in dem ein elektronischer Aufbau betrieben werden kann.

In dem kritischen Bereich soll beispielsweise die elektrische Kapazität, also die Messkapazität, einer Probe gemessen werden. Dies kann die Messung der Permittivität oder der elektrische Leitfähigkeit einer Probe beinhalten oder die Messung eines Füllstandes. Die Messung kann kabellos erfolgen, sodass im Anregungskreis Standardelektronik trotz ungünstiger Umweltbedingungen eingesetzt werden kann.

Dies wird dadurch erreicht, dass die Messanordnung einen Transformator umfasst. Die Primärseite des Transformators mit der Auswerteeinrichtung wird ausserhalb des kritischen Bereiches betrieben. Die Sekundärseite mit dem Kapazitätssensor befindet sich beispielsweise im kritischen Bereich. Die Sekundärseite kann somit ohne aktive elektronische Bauteile realisiert werden.

Die Sekundärseite kann beispielsweise mit einem Anregungssignal mit wechselnder Frequenz angesteuert werden. Beispielsweise wird dazu ein ganzes Frequenzband kontinuierlich abgefahren. Die Messanordnung kann jedoch auch durch einen selbst auf der Resonanz schwingenden Oszillator verwirklicht werden. Eine weitere Möglichkeit besteht darin, einen Puls oder Schritt zu erzeugen, und die hierauf erfolgte Antwort zu messen. Durch eine Impedanzbestimmung auf der Primärseite können die Resonanzfrequenz des Transformators und die Güte bestimmt werden.

Der Schwingkreis kann jedoch auch auf der Primärseite und der Anregungskreis kann auf der Sekundärseite des Transformators angeordnet werden.

Die Erfindung betrifft gemäß einem Aspekt eine Messanordnung zum Bestimmen einer Messkapazität einer Messprobe, umfassend einen Schwingkreis umfassend einen Kapazitätssensor und eine erste Induktivität, welche mit dem Kapazitätssensor elektrisch verbunden ist, und einen Anregungskreis umfassend eine zweite Induktivität, welche mit der ersten Induktivität magnetisch koppelbar ist, um ein Anregungssignal zum Überführen des Schwingkreises in einen Resonanzbetrieb zu übertragen, einen Anregungssignalgenerator, welcher mit der zweiten Induktivität elektrisch verbunden und ausgebildet ist, das Anregungssignal zu erzeugen, und eine Auswerteeinrichtung, welche ausgebildet ist, die Messkapazität aus einer in dem Resonanzbetrieb erfassbaren elektrischen Größe zu bestimmen. Die erste Induktivität bildet gemäß einer Ausführungsform eine Sekundärspule, und die zweite Induktivität bildet eine Primärspule eines Transformators.

Dadurch wird beispielsweise der technische Vorteil erreicht, dass eine physikalische Größe über eine Entfernung hinweg kontaktlos gemessen werden kann. Besonders vorteilhaft kann der Schwingkreis in einer Umgebung verwendet werden, in der kein Kabelanschluss möglich ist.

Der Schwingkreis ist gemäß einer Ausführungsform passiv, d.h. er umfasst keine eigenständige Energiequelle. Die Versorgung des Schwingkreises mit elektrischer Energie erfolgt beispielsweise ausschließlich über die magnetische Feldkopplung zwischen der ersten Induktivität, welche eine Spule sein kann, und der zweiten Induktivität, welche in unmittelbarer Nähe der Induktivität zur Erzeugung der magnetischen Feldkopplung gebracht werden kann.

Gemäß einer Ausführungsform ist die erste Induktivität mit elektrischen Anschlüssen des Kapazitätssensors elektrisch verbunden. Dadurch wird beispielsweise der technische Vorteil erreicht, dass sich das über die erste Induktivität empfangene Anregungssignal direkt in den Kapazitätssensor einkoppeln lässt.

Gemäß einer Ausführungsform umfasst der Kapazitätssensor voneinander beabstandete metallische Messelektroden zum Wechselwirken mit der Messprobe.

Gemäß einer Ausführungsform ist ein zylindrischer Behälter zum Aufnehmen der Messprobe, insbesondere einer Messflüssigkeit, vorgesehen, wobei die Messelektroden übereinander an dem zylindrischen Behälter angeordnet, insbesondere jeweils ringförmig den zylindrischen Behälter umschließend, angeordnet sind. Der zylindrische Behälter kann beispielsweise ein Reagenzglas sein.

Gemäß einer Ausführungsform umfasst die Messanordnung eine Zentrifuge, in welche der zylindrische Behälter einsetzbar ist.

Gemäß einer Ausführungsform ist die Zentrifuge ausgebildet, den zylindrischen Behälter um eine außerhalb des zylindrischen Behälters liegende Rotationsachse zu bewegen bzw. zu rotieren. So kann die erste Induktivität an der zweiten Induktivität vorbei rotieren.

Gemäß einer Ausführungsform ist die Zentrifuge ausgebildet ist, den zylindrischen Behälter um eine außerhalb des zylindrischen Behälters liegende Rotationsachse zu rotieren, wobei die erste Induktivität mit dem zylindrischen Behälter verbunden ist und eine Rotationsachse der ersten Induktivität mit der Mittelachse der zweiten Induktivität zusammenfällt, so dass der Kopplungsfaktor der ersten Induktivität mit der zweiten Induktivität über eine Zeit konstant bleibt.

Gemäß einer Ausführungsform ist die Auswerteeinrichtung ausgebildet, eine Stromamplitude in dem Anregungskreis, eine Spannungsamplitude in dem Anregungskreis, eine Frequenz, insbesondere eine Eigenfrequenz, eine elektrische Dämpfung, oder eine elektrische Impedanz an den elektrischen Anschlüssen der zweiten Induktivität als die elektrische Größe zu bestimmen.

Gemäß einer Ausführungsform ist die Auswerteeinrichtung ausgebildet, einen Maximalwert oder einen Minimalwert der elektrischen Größe zu erfassen, um den Resonanzbetrieb zu detektieren.

Gemäß einer Ausführungsform ist der Anregungssignalgenerator ausgebildet, das Anregungssignal mit wechselnder Frequenz zu erzeugen, wobei die Auswerteeinrichtung ausgebildet ist, eine elektrische Impedanz an den elektrischen Anschlüssen der zweiten Induktivität als die elektrische Größe zu bestimmen.

Gemäß einer Ausführungsform ist der Anregungssignalgenerator ausgebildet, einen Puls als das Anregungssignal zu erzeugen, wobei die Auswerteeinrichtung ausgebildet ist, eine Schwingfrequenz oder eine elektrische Dämpfung als die elektrische Größe zu bestimmen.

Gemäß einer Ausführungsform ist der Anregungssignalgenerator ausgebildet, im Resonanzbetrieb eine Stromamplitude oder eine Spannungsamplitude als die elektrische Größe zu bestimmen.

Gemäß einer Ausführungsform ist der Anregungssignalgenerator ausgebildet, die Messkapazität anhand einer Zuordnung der im Resonanzbetrieb erfassten elektrischen Größe zu einer Messkapazität, insbesondere anhand einer Look-Up-Tabelle, zu bestimmen.

Gemäß einer Ausführungsform ist der Anregungssignalgenerator ausgebildet, das Anregungssignal mit der Resonanzfrequenz oder ein Rauschsignal als das Anregungssignal zum Überführen des Schwingkreises in den Resonanzbetrieb zu erzeugen.

Gemäß einer Ausführungsform sind die erste Induktivität und die zweite Induktivität galvanisch getrennt und sie bilden bei magnetischer Kopplung einen elektrischen Übertrager.

Gemäß einem weiteren Aspekt betrifft die Erfindung die Verwendung der erfindungsgemäßen Messanordnung zur Messung einer Messkapazität einer Messprobe, mit Überführen des Schwingkreises in einen Resonanzbetrieb durch Erzeugen des Anregungssignals, Erfassen einer elektrischen Größe in dem Resonanzbetrieb, und Bestimmen der Messkapazität anhand der erfassten elektrischen Größe.

Gemäß einer Ausführungsform der Messanordnung ist der Sensor ein Kapazitätssensor, der mit der Induktivität einen Resonanzkreis formt. Dadurch wird beispielsweise der Vorteil erreicht, dass die physikalische Größe über eine Resonanzfrequenz oder Impedanz des Schwingkreises bestimmt werden kann.

Gemäß einer Ausführungsform umfasst die jeweilige Induktivität einen metallischen Spulenkern. Dadurch wird beispielsweise der Vorteil erreicht, dass sich eine Resonanzfrequenz des Schwingkreises anpassen lässt.

Weitere Ausführungsbeispiele der Erfindung sind in den Zeichnungen dargestellt und werden im Folgenden näher beschrieben.

Es zeigen:

Fig. 1     eine schematische Darstellung einer Messanordnung;

Fig. 2     eine schematische Darstellung einer Messanordnung;

Fig. 3       einen Messwandler;

Fig. 4       ein Ersatzschaltbild des in Fig. 3 dargestellten Messwandlers;

Fig. 5       eine Messvorrichtung;

Fig. 6       eine schematische Darstellung einer Messanordnung;

Fig. 7       eine perspektivische Darstellung einer Messanordnung;

Fig. 8       ein Frequenzspektrum;

Fig. 9       ein Ablaufdiagramm des Messverfahrens, und

Fig. 10      ein Ablaufdiagramm des Auswerteverfahrens.

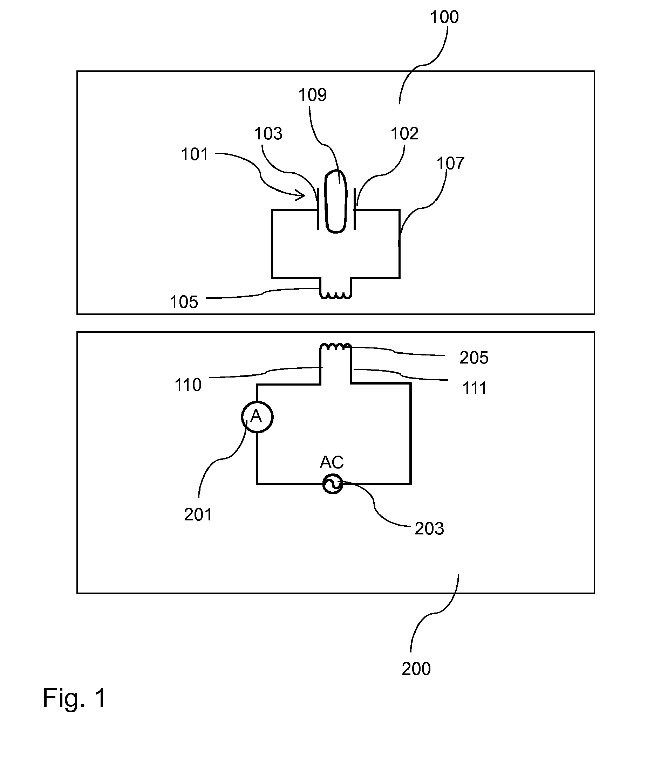

Fig.1 zeigt eine schematische Darstellung einer ersten Ausführungsform einer Messanordnung.

Die Messanordnung umfasst einen Schwingkreis 100 mit einem Kapazitätssensor 101 zum Erfassen einer physikalischen Größe, der durch zwei Elektroden 102 und 103 gebildet wird, sowie einer ersten Induktivität 105, welche eine Sekundärspule eines Transformators bildet. Der Schwingkreis 100 kann beispielsweise in einem kritischen Bereich angeordnet sein, in dem eine kontaktbehaftete Messung nur schwer möglich ist. Aufwändige Aufbauten und elektronische Schaltungen können in einem kritischen Bereich nicht untergebracht werden und ein Kontakt zum Anschluss von Kabeln kann nicht zur Verfügung stehen.

Zwischen den beiden Elektroden 102 und 103 ist beispielsweise ein Messobjekt 109 angeordnet, dessen Kapazität als Messkapazität bestimmt werden soll. Bei dem Messobjekt 109 handelt es sich beispielsweise um ein Reagenzglas mit einer Blutprobe. Im Allgemeinen können jedoch auch andere Messobjekte 109 verwendet werden.

Die Messanordnung umfasst lediglich passive, elektrische Bauteile.

In dem Schwingkreis 100 wird Energie zwischen dem magnetischen Feld der ersten Induktivität 105 und dem elektrischen Feld des Kapazitätssensors101 periodisch ausgetauscht, so dass abwechselnd eine Stromstärke oder eine Spannung vorliegen. Die Frequenz, mit der sich dieses im ungestörten Fall periodisch wiederholt, ist durch die Formel gemäß der Schwingkreisgleichung bzw. der Thomsonschen Schwingungsgleichung

$$f_0 = \frac{1}{2\pi\sqrt{LC}} \tag{1}$$

für die Resonanzfrequenz $f_0$ gegeben, in der L den Wert der Induktivität und C den Wert der Kapazität angibt. Verändert sich die Induktivität L oder die Kapazität C, so ändert sich die Resonanzfrequenz $f_0$. Optional kann zusätzlich ein Kopplungsfaktor berücksichtig werden.

Der Schwingkreis 100 kann durch eine induktive, magnetische Kopplung zu Schwingungen angeregt werden. Zu diesem Zweck wird in die erste Induktivität 105 ein elektrisches Sensoranregungssignal induziert, das den Schwingkreis 100 zu Schwingungen anregt.

Wird der Schwingkreis 100 durch einen Schaltvorgang oder einen Impuls einmalig angestoßen, dann führt er freie Schwingungen mit der Resonanzfrequenz $f_0$ (Eigenschwingungen) aus, die aufgrund von elektrischen Verlusten abklingen. Wird der Schwingkreis 100 jedoch periodisch erregt, dann führt er nach Ablauf der Einschwingzeit periodische erzwungene Schwingungen aus, deren Frequenz identisch mit der Erregerfrequenz ist. Die hierbei auftretenden Resonanzerscheinungen können zur Messung der Messobjekts 109 verwendet werden. Je nach Frequenz der Anregung weist der Schwingkreis 100 daher eine andere Impedanz auf.

Über die erste Induktivität 105 kann ein Sensorerfassungssignal des Kapazitätssensors 101 ausgelesen bzw. ausgegeben werden, das eine physikalische Größe der Probe widerspiegelt. In diesem Fall wird das Sensorerfassungssignal durch die Kapazität beeinflusst, die durch die beiden Elektroden 102 und 103 und die Messprobe 109 beeinflusst wird. Um die Resonanzfrequenz des Schwingkreises 100 an eine gewünschte Frequenz anzupassen, kann die erste Induktivität 105 einen Spulenkern umfassen.

Die Messanordnung umfasst ferner einen Anregungskreis 200 mit einer zweiten Induktivität 205 mit Anschlüssen 110, 111, welche wie die erste Induktivität 105 als Spule gebildet sein kann. Die zweite Induktivität 205 bildet gemäß einer Ausführungsform eine Primärspule des Transformators. Der Anregungskreis 200 umfasst eine Auswertungseinrichtung 201, wie beispielsweise ein Strommessgerät, durch das der zu messende Strom fließt. Weiter umfasst die Sensorauswertevorrichtung 200 einen Anregungssignalgenerator 203, welcher ein Frequenz- oder Funktionsgenerator ist oder durch einen durchstimmbaren Oszillator gebildet sein und eine Wechselspannung mit einer oder mehreren Frequenzen erzeugen kann. Die zweite Induktivität 205 erzeugt in diesem Fall ein magnetisches Wechselfeld, das von der ersten Induktivität 105 empfangen wird. Die erste Induktivität 105 und die zweite Induktivität 205 sind induktiv gekoppelt und es wird ein Transformator gebildet. Eine Auswerteelektronik ist beispielsweise über ein Kabel mit der Spule 205 verbunden.

Die erste Induktivität 105 wird beispielsweise mit einer Frequenzrampe mit dem Anregungssignalgenerator 203 angesteuert. Die Auswertungseinrichtung 201 misst dann einen geringeren Strom, wenn der Messkreis in Resonanz mit der Anregungsfrequenz ist. Aus der so bestimmten Resonanzfrequenz kann die Kapazität zwischen den Elektroden 102 und 103 bestimmt werden. Die Güte des Schwingkreises 100 kann durch die Stromstärke ermittelt werden.

Eine direkte elektrische Verbindung der ersten Induktivität 105 und der zweiten Induktivität 205 ist nicht erforderlich, so dass sich der Schwingkreis 100 auch kontaktlos über eine gewisse Entfernung anregen lässt. Die zweite Induktivität 205 der Sensorauswertevorrichtung kann beispielsweise in einer Entfernung bis zu einigen Spulendurchmessern von der ersten Induktivität 105 im Schwingkreis 100, d.h. der Messkopfinduktivität, angeordnet sein.

Dadurch lässt sich beispielsweise die Kapazität C der Messprobe 109 in einer Umgebung ermitteln, in der kein Kabelanschluss möglich ist und durch widrige Umgebungsbedingungen der Einsatz von Elektronik erschwert ist.

Der Schwingkreis 100 kann einseitig geerdet sein. Beispielsweise kann die Elektrode 102 geerdet sein. Hierzu kann die Elektrode 102 beispielsweise durch eine metallische Wand eines Stahltanks gebildet werden.

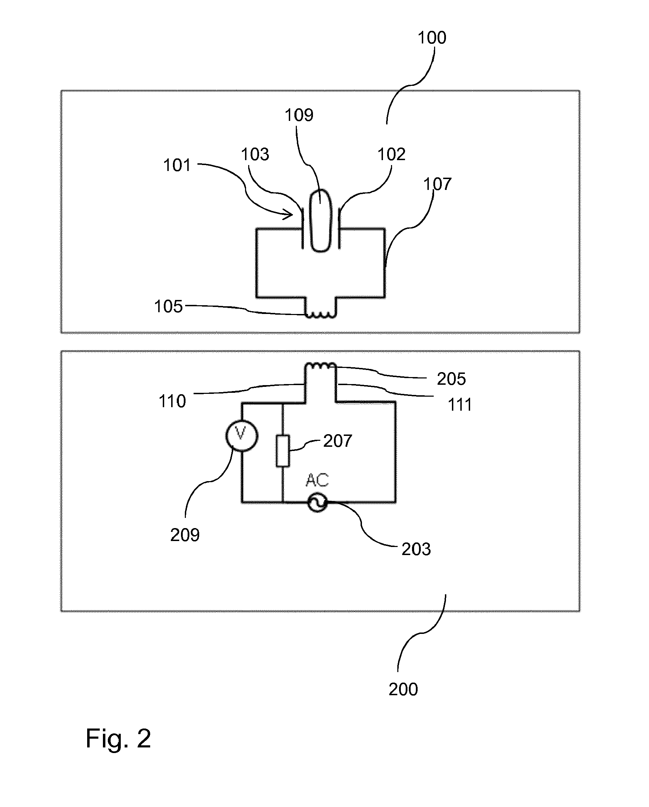

Fig. 2 zeigt eine schematische Darstellung einer zweiten Ausführungsform der Messanordnung. Diese Ausführungsform unterscheidet sich von der in Fig.1 gezeigten Ausführungsform, dass in dem Anregungskreis 200 ein Widerstand 207 und ein Spannungsmesser 209 als Auswerteeinrichtung in Serie geschaltet ist.

Sind die Induktivitätswerte der ersten Induktivität 105 und der zweite Induktivität 205 im Primär- und Sekundärkreis identisch und durch L gegeben, errechnet sich die gemessene Kapazität $C_{mess}$ zu

$$C_{mess} = \frac{1}{L(2\pi\, f_0)^2} \qquad\qquad (2)$$

Die Resonanzfrequenz $f_0$ ist dann erreicht, wenn die Spannung am Spannungsmesser 209 minimal ist.

Die Messanordnung kann vielseitig verwendet werden, wie beispielsweise im Bereich der Hochtemperatur-Sensorik, der Messung an und in bewegten Teilen, bei im Körper implantierten Sensoren oder zur Füllstandsmessung in einem Tank. Im Allgemeinen kann die induktiv mit der Sensorauswertungsvorrichtung gekoppelte Messanordnung zur Messung einer Kapazität, Induktivität oder Leitfähigkeit dienen.

Die Ankopplung der Messanordnung an die Sensorauswertevorrichtung erfolgt über ein magnetisches Feld, das die Messanordnung im Resonanzfall gegenphasig "zurückspiegelt". Durch diese Art der Kopplung kann eine Untersuchungsfrequenz gewählt werden, die tiefer liegt als im Fall von Mikrowellen. Auf eine aufwändige Elektronik zum Erzeugen und Auswerten von Mikrowellensignalen kann daher verzichtet werden.

Gemäß einer Ausführungsform kann die Sekundärspule 105 im kritischen Bereich angeordnet werden - sie bildet mit den Messelektroden 102 und 103 den Schwingkreis 100. Die Resonanz und die Güte des Schwingkreises 100 hängen von der Messprobe 109 ab. Durch eine transformatorische Kopplung wird die Impedanz des Sekundärkreises auf den Primärkreis übertragen und dort gemessen.

Gemäß einer Messemethode erzeugt der Anregungssignalgenerator 203, beispielsweise ein Frequenzgenerator eine Wechselspannung mit einer von der Zeit abhängigen Frequenz. Wenn der Sekundärkreis in Resonanz gerät, vermindert sich der Strombedarf im Primärkreis. Dies kann beispielsweise mit dem Strommesser 201 gemessen werden.

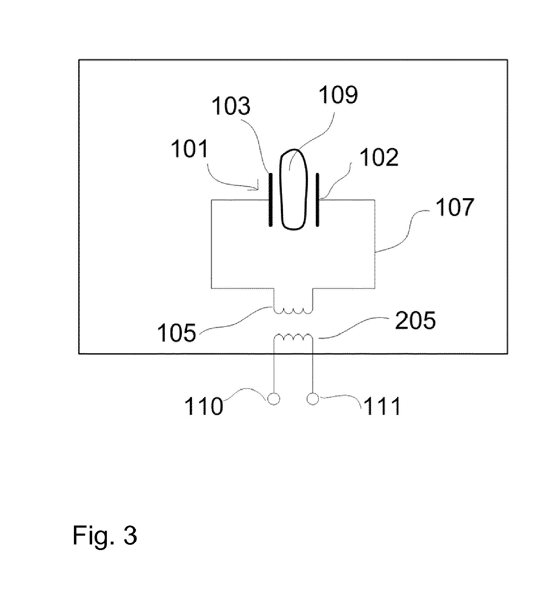

Der Schwingkreis 100 sowie die zweite Induktivität 205 können zu einem Messwandler zusammengefasst werden, wie es in Fig. 3 dargestellt ist. Der Messwandler wird gebildet durch einen Schwingkreis, bestehend aus der Messkapazität 101 mit der Messprobe 109 und der Induktivität 105 und einer Induktivität 205 welche durch magnetische Kopplung mit der Induktivität 105 verbunden ist. Die magnetische Kopplung dient der kontaktlosen Übertragung der Impedanz des Sekundären Schwingkreises mit dem Messkondensator 109 auf das Messsystem der Primärseite.

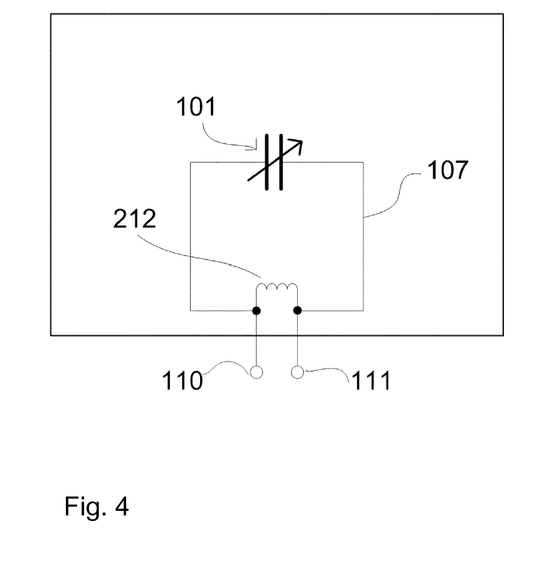

Fig. 4 zeigt ein vereinfachtes Ersatzschaltbild des in Fig. 3 dargestellten Messwandlers. Das Ersatzschaltbild basiert auf den Annahmen, dass die erste Induktivität 105 und die zweite Induktivität 205 gleich sind und dass die Kopplung zwischen der ersten Induktivität 105 und der zweiten Induktivität 205 Eins ist. An den Anschlüssen 110 und 111 wird gemäß einer Ausführungsform eine komplexe Impedanz anstehen in Abhängigkeit der Frequenz eines Anregungssignals und in Abhängigkeit der Kapazität des Kapazitätssensors 101 mit der Messprobe 109.

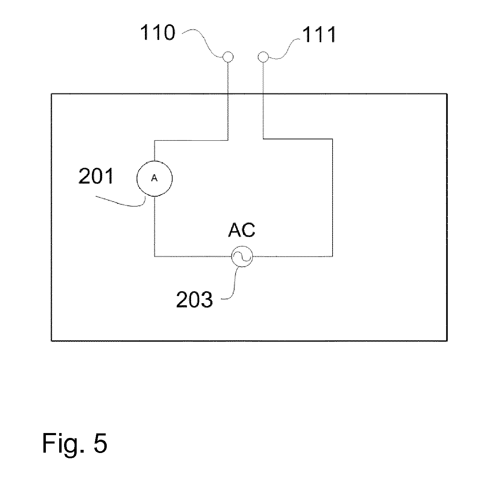

Der verbleibende Teil des Anregungskreises 200 ohne die zweite Induktivität 205 kann als eine Messvorrichtung aufgefasst werden, deren Blockdiagramm in Fig. 5 dargestellt ist.

Die Messvorrichtung umfasst den Anregungssignalgenerator 203, beispielsweise einen Oszillator oder einen Pulsgenerator, und die Auswerteeinrichtung 201 als elektrische Messeinheit zur Strom- oder Spannungsmessung. Damit kann die komplexe Impedanz des Messwandlers in Abhängigkeit der Frequenz bestimmt werden. Es kann auch die Dämpfung und die Frequenz eines frei schwingenden Oszillators gemessen werden.

Das Anregungssignal kann mittels des Anregungssignalgenerators 203 beispielsweise mit wechselnder Frequenz erzeugt werden, wobei die Impedanz, beispielsweise über eine Strom- oder Spannungsmessung gemessen wird.

Gemäß einer Ausführungsform kann der in Fig. 3 dargestellte Messwandler auf der Resonanzfrequenz des Sensorsystems schwingen und es wird Frequenz und Strom- oder Spannungsamplitude gemessen. Der Messwandler kann somit ein Teil eines Oszillators sein, so dass dessen Frequenz und Amplitude durch die Messgröße festgelegt wird. Hierfür eignet sich zum Beispiel ein Meissner Oszillator.

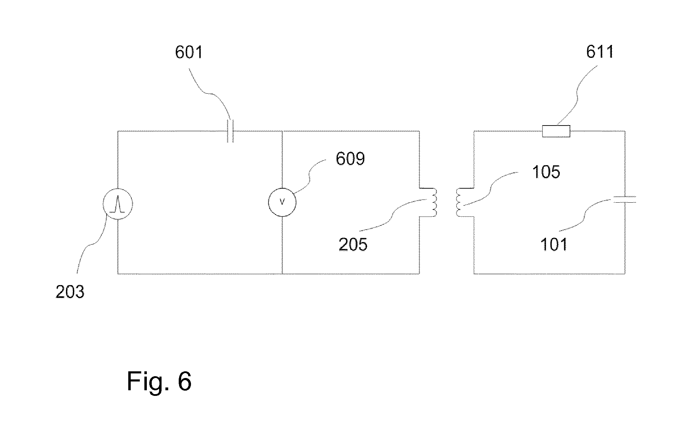

Der Anregungssignalgenerator 203 kann gemäß einer Ausführungsform einen Schritt oder einen Puls ausgeben und es wird die Schritt bzw. Pulsantwort gemessen, woraus die Eigenfrequenz und die Dämpfung bestimmt werden können. Der Puls kann den Schwingkreis anregen, welcher dann ausschwingt. Eine realisierbare Schaltung einer Messanordnung ist in Fig. 6 dargestellt.

Die Messanordnung umfasst im Primärkreis zusätzlich eine Kapazität 601, beispielsweise 1 pF. Der Anregungssignalgenerator 203 kann beispielsweise Spannungspulse erzeugen. Der Kapazitätssensor 101 im Sekundärkreis kann aufgrund der Elektroden einen Kapazitätswert von 1 pF umfassen. Der Sekundärkreis kann ferner einen Widerstand 611 aufweisen, welcher zusammen mit der Kapazität 101 die Impedanz der Messprobe abbildet.

Der Anregungssignalgenerator 203 erzeugt einen Puls. Durch die Kapazität 601 wird der Anregungssignalgenerator 203 abgekoppelt und schwingt bis auf die Beeinflussung durch die Kapazität 601 frei aus. Die Messkapazität kann anhand des Ausschwingverhaltens, insbesondere der Ausschwingfrequenz, bestimmt werden. Der ohmsche Anteil 611 der Messprobe kann mit der Ausschwingzeitdauer bestimmt werden. Hierzu können empirisch bestimmte Werte in einer Look-Up-Tabelle zusammengefasst werden.

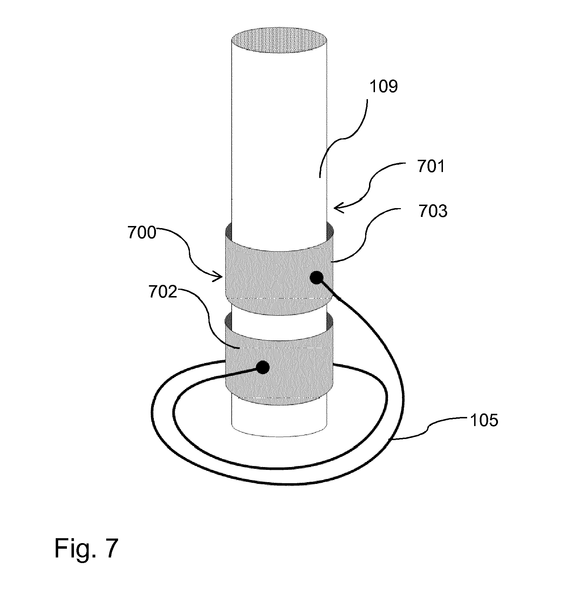

Fig. 7 zeigt eine perspektivische Darstellung einer Messanordnung, die beispielsweise zur Permittivitätsmessung einer Flüssigkeit verwendet werden kann. Beispielsweise kann mithilfe einer derartigen Messung die Trennung von Blut in einer Zentrifuge bestimmt werden.

Die Messanordnung umfasst einen Kapazitätssensor 700 mit zwei übereinander liegenden Elektroden 702, 703. Die Elektroden 702, 703 sind beispielsweise ringförmig ausgeführt.

Die Messanordnung umfasst ferner einen zylindrischen Behälter 701, beispielsweise ein Reagenzglas zum Aufnehmen des Messobjektes 109, beispielsweise einer Flüssigkeit. Der zylindrische Behälter 701 kann mit dem Messobjekt 109 gemäß einer Ausführungsform ein gemeinsames Messobjekt bilden, dessen Kapazität bestimmt wird.

Der zylindrische Behälter 701 ist beispielsweise in eine Zentrifuge einsetzbar. Die Zentrifuge stellt gemäß einer Ausführungsform einen kritischen Bereich in oben genanntem Sinne dar, in dem eine direkte Kontaktierung der Messanordnung nur schwer möglich ist. Die zwei ringförmigen Elektroden 702 und 703 sind um das stabförmige Reagenzglas herum angeordnet und dienen der Kapazitätsmessung. Die Elektroden 702 und 703 sind mit der als Spule ausgeführten ersten Induktivität 105 elektrisch verbunden. Der so gebildete Schwingkreis 100 arbeitet somit ohne eine leitungsgebundene Energieversorgung.

Der Anregungskreis 200 ist beispielsweise an einem geeigneten Ort innerhalb der Zentrifuge und außerhalb des rotierenden Teils angeordnet. Bewegt sich das Reagenzglas mit dem Schwingkreis 100 an dem Anregungskreis 200 vorbei, kann kontaktlos ein Kapazitätswert der Flüssigkeit in dem zylindrischen Behälter 701 bestimmt werden.

Durch Zentrifugieren kann Blut in einen zellulären Bestandteil und einen flüssigen Teil aufgeteilt werden, in dem die Zellen "schwimmen". Der flüssige, zellfreie Teil wird als Blutplasma bezeichnet. Vor einem Zentrifugieren erzeugt das ungetrennte Blut zwischen den beiden Ringelektroden 702 und 703 beispielsweise eine erste Kapazität im Schwingkreis 107. Sobald sich die beiden Blutkomponenten voneinander getrennt haben und sich im Bereich zwischen den Ringelektroden 702 und 703 Blutplasma überwiegend befindet, wird durch die geänderte Dielektrizitätszahl eine zweite Kapazität im Schwingkreis 100 erzeugt. Je nach Resonanzfrequenz lässt sich daher bestimmen, ob sich der zellulärer Bestandteil und der flüssigen Teil des Blutes getrennt haben.

Die Ringelektroden 702 und 703 können verschiebbar an dem Reagenzglas 701 angeordnet sein, so dass diese je nach Füllstand eingestellt werden können.

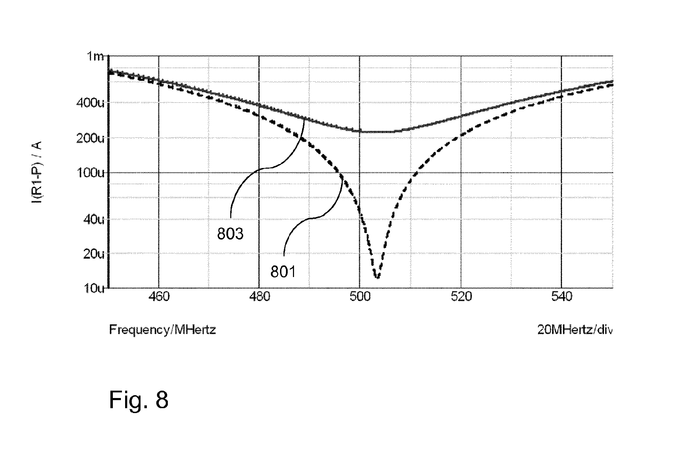

Fig. 8 zeigt ein Diagramm einer Strommessung über die Frequenz. Die Kurve 801 zeigt eine Probe, die eine Messkapazität von 1 pF verursacht, während die Kurve 803 ebenfalls eine Probe zeigt, die eine Messkapazität von 1 pF verursacht, jedoch mit einem erhöhten Serienwiderstand. Bei höheren Messkapazitäten verringert sich die Resonanzfrequenz.

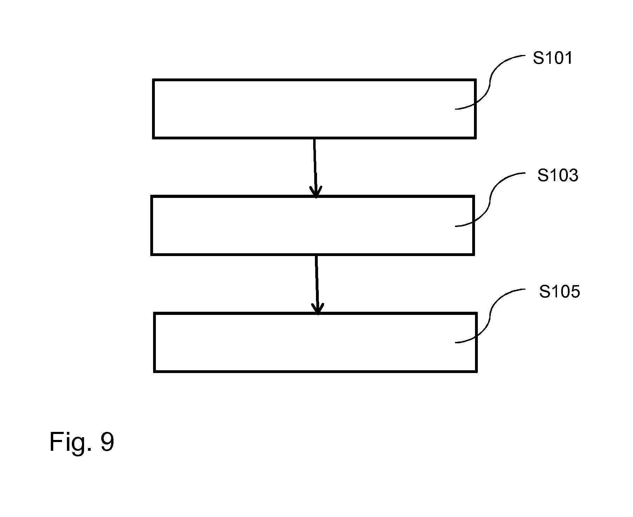

Fig. 9 zeigt ein Ablaufdiagramm des erfindungsgemäßen Messverfahrens. Im Schritt S 101 wird durch den Schwingkreis 100 ein Anregungssignal mittels eines magnetischen Koppelfeldes unter Verwendung einer ersten Induktivität 105 empfangen. Im Schritt S103 wird durch den Kapazitätssensor 101 eine Kapazität erfasst und ein Erfassungssignal ausgegeben. Im Schritt S105 wird das Erfassungssignal mittels des magnetischen Koppelfeldes unter Verwendung der ersten Induktivität 105 ausgegeben.

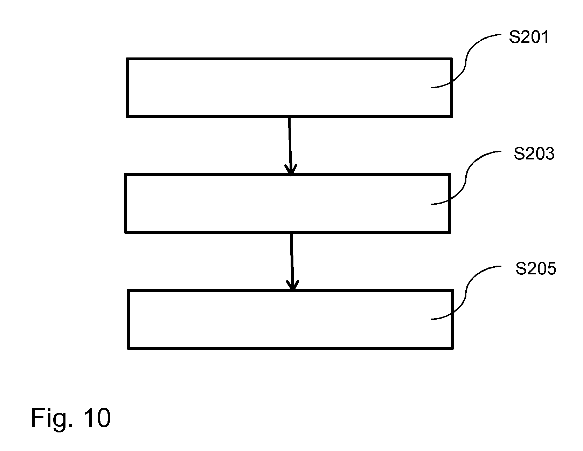

Fig. 10 zeigt ein Ablaufdiagramm des Sensorauswerteverfahrens in dem Anregungskreis 200. Im Schritt S201 wird ein Anregungssignals mittels eines magnetischen Koppelfeldes unter Verwendung der zweiten Induktivität 205 übertragen, um den Kapazitätssensor 101 zum Erfassen einer physikalischen Größe anzuregen. Im Schritt S203 wird ein Erfassungssignal von dem Kapazitätssensor 101 mittels eines magnetischen Koppelfeldes unter Verwendung der zweiten Induktivität 205 empfangen. Im Schritt S205 wird eine die Messkapazität wie vorstehend ausgeführt auf der Basis des Erfassungssignals bestimmt.

**Patentansprüche**

1. Messanordnung zum Bestimmen einer Messkapazität einer Messprobe, umfassend:

   einen Schwingkreis (100) umfassend einen Kapazitätssensor (101) und eine erste Induktivität (105), welche mit dem Kapazitätssensor (101) elektrisch verbunden ist; und
   einen Anregungskreis (200) umfassend eine zweite Induktivität (205), welche mit der ersten Induktivität (105) magnetisch koppelbar ist, um ein Anregungssignal zum Überführen des Schwingkreises (100) in einen Resonanzbetrieb zu übertragen, einen Anregungssignalgenerator (203), welcher mit der zweiten Induktivität (205) elektrisch verbunden und ausgebildet ist, das Anregungssignal zu erzeugen, und eine Auswerteeinrichtung (201), welche ausgebildet ist, die Messkapazität aus einer in dem Resonanzbetrieb erfassbaren elektrischen Größe zu bestimmen.

2. Messanordnung (100) nach Anspruch 1, wobei die erste Induktivität (105) mit elektrischen Anschlüssen des Kapazitätssensors (101) elektrisch verbunden ist.

3. Messanordnung (100) nach einem der vorstehenden Ansprüche, wobei der Kapazitätssensor (101) voneinander beabstandete metallische Messelektroden (102, 103, 702, 703) zum Wechselwirken mit der Messprobe umfasst.

4. Messanordnung (100) nach einem der vorstehende Ansprüche, umfassend einen zylindrischen Behälter (701) zum Aufnehmen der Messprobe, insbesondere einer Messflüssigkeit, wobei die Messelektroden (702, 703)) übereinander an dem zylindrischen Behälter (701), insbesondere jeweils ringförmig den zylindrischen Behälter umschließend, angeordnet sind.

5. Messanordnung (100) nach Anspruch 4, umfassend eine Zentrifuge, in welche der zylindrische Behälter (701) einsetzbar ist.

6. Messanordnung (100) nach Anspruch 5, wobei die Zentrifuge ausgebildet ist, den zylindrischen Behälter (701) um eine außerhalb des zylindrischen Behälters (701) liegende Rotationsachse zu rotieren, wobei die erste Induktivität (105) an der zweiten Induktivität (205) vorbei rotierbar ist.

7. Messanordnung (100) nach Anspruch 5, wobei die Zentrifuge ausgebildet ist, den zylindrischen Behälter (701) um eine außerhalb des zylindrischen Behälters (701) liegende Rotationsachse zu rotieren, wobei die erste Induktivität (105) mit dem zylindrischen Behälter (701) verbunden ist und eine Rotationsachse der ersten Induktivität (105) mit der Mittelachse der zweiten Induktivität (205) zusammenfällt, so dass der Kopplungsfaktor der ersten Induktivität (105) mit der zweiten Induktivität (205) über eine Zeit konstant bleibt.

8. Messanordnung (100) nach einem der der vorstehende Ansprüche, wobei die Auswerteeinrichtung (201) ausgebildet ist, eine Stromamplitude in dem Anregungskreis (200), eine Spannungsamplitude in dem Anregungskreis (200), eine Frequenz, insbesondere eine Eigenfrequenz, eine elektrische Dämpfung, oder eine elektrische Impedanz an den elektrischen Anschlüssen (110, 111) der zweiten Induktivität (205) als die elektrische Größe zu bestimmen.

9. Messanordnung (100) nach einem der der vorstehende Ansprüche, wobei die Auswerteeinrichtung (201) ausgebildet ist, einen Maximalwert oder einen Minimalwert der elektrischen Größe zu erfassen, um den Resonanzbetrieb zu detektieren.

10. Messanordnung (100) nach einem der der vorstehende Ansprüche, wobei der Anregungssignalgenerator (201) ausgebildet ist, das Anregungssignal mit wechselnder Frequenz zu erzeugen, wobei die Auswerteeinrichtung (203) ausgebildet ist, eine elektrische Impedanz an den elektrischen Anschlüssen (110, 111) der zweiten Induktivität (205) als die elektrische Größe zu bestimmen.

**11.** Messanordnung (100) nach einem der der vorstehende Ansprüche, wobei der Anregungssignalgenerator (203) ausgebildet ist, einen Puls als das Anregungssignal zu erzeugen, und wobei die Auswerteeinrichtung (201) ausgebildet ist, eine Schwingfrequenz oder eine elektrische Dämpfung als die elektrische Größe zu bestimmen.

**12.** Messanordnung (100) nach einem der der vorstehende Ansprüche, wobei der Anregungssignalgenerator (203) ausgebildet ist, im Resonanzbetrieb eine Stromamplitude oder eine Spannungsamplitude als die elektrische Größe zu bestimmen.

**13.** Messanordnung (100) nach einem der der vorstehende Ansprüche, wobei der Anregungssignalgenerator (203) ausgebildet ist, die Messkapazität anhand einer Zuordnung der im Resonanzbetrieb erfassten elektrischen Größe zu einer Messkapazität, insbesondere anhand einer Look-Up-Tabelle, zu bestimmen.

**14.** Messanordnung (100) nach einem der der vorstehende Ansprüche, wobei der Anregungssignalgenerator (203) ausgebildet ist, das Anregungssignal mit der Resonanzfrequenz oder ein Rauschsignal als das Anregungssignal zum Überführen des Schwingkreises (100) in den Resonanzbetrieb zu erzeugen.

**15.** Verwendung der Messanordnung gemäß einem der Ansprüche 1 bis 14 zur Messung einer Messkapazität einer Messprobe, mit:

a) Überführen des Schwingkreises (100) in einen Resonanzbetrieb durch Erzeugen des Anregungssignals;
b) Erfassen einer elektrischen Größe in dem Resonanzbetrieb; und
c) Bestimmen der Messkapazität anhand der erfassten elektrischen Größe.

Fig. 1

Fig. 2

Fig. 3

Fig. 4

Fig. 5

Fig. 6

Fig. 7

Fig. 8

S101

S103

S105

Fig. 9

S201

S203

S205

Fig. 10

Europäisches
Patentamt

European
Patent Office

Office européen
des brevets

# EUROPÄISCHER RECHERCHENBERICHT

Nummer der Anmeldung

EP 12 18 4464

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | US 4 187 462 A (HAKER ROLF [DE] ET AL) 5. Februar 1980 (1980-02-05) * Zusammenfassung; Abbildungen 1,3,4 * * Spalte 6, Zeile 1 - Spalte 8, Zeile 62 * ----- | 1-15 | INV. G01N27/22 |
| X | WO 92/18837 A1 (FLUIDRIVE ENG CO LTD [GB]) 29. Oktober 1992 (1992-10-29) * Zusammenfassung; Abbildungen 1,2 * * Seite 6, Absatz 2 - Seite 11, letzter Absatz * ----- | 1,2,8, 13,15 | |
| X | GB 2 248 301 A (ICI PLC [GB]) 1. April 1992 (1992-04-01) * Zusammenfassung; Abbildungen 2,5,6 * * Seite 8, Absatz 6 - Seite 10, letzter Absatz * * Seite 4, Absatz 4 - Seite 5, Absatz 3 * ----- | 1-5,9, 11,15 | |
| Y | DE 15 73 236 A1 (HITACHI LTD) 10. Februar 1972 (1972-02-10) * Seite 1, Absatz 1 - Seite 2, Absatz 1; Abbildungen 1-5 * * Seite 4, Absatz 2 - Seite 5, Absatz 1 * * Seite 6, Absatz 2 - Seite 12, Absatz 2 * ----- | 1-15 | |
| X | DE 101 27 978 C1 (VOGT ELECTRONIC AG [DE]) 14. November 2002 (2002-11-14) * Zusammenfassung; Abbildungen 1,2 * * Absatz [0012] - Absatz [0019] * ----- | 1-3, 8-10, 12-15 | RECHERCHIERTE SACHGEBIETE (IPC) G01N |
| A | DE 37 09 665 A1 (KASTL HEINER [DE]; OHLENSCHLAEGER GERHARD [DE]) 6. Oktober 1988 (1988-10-06) * Zusammenfassung; Abbildungen 3a-3d * ----- | 1-15 | |
| A | GB 2 260 407 A (BARNES CHRISTOPHER [GB]) 14. April 1993 (1993-04-14) * Zusammenfassung; Abbildungen 1,2 * ----- | 1-15 | |

Der vorliegende Recherchenbericht wurde für alle Patentansprüche erstellt

| Recherchenort | Abschlußdatum der Recherche | Prüfer |
|---|---|---|
| Berlin | 13. März 2013 | Ernst, Monika |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.......................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**                    EP 12 18 4464

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

13-03-2013

| Im Recherchenbericht angeführtes Patentdokument | | Datum der Veröffentlichung | Mitglied(er) der Patentfamilie | | Datum der Veröffentlichung |
|---|---|---|---|---|---|
| US 4187462 | A | 05-02-1980 | DE | 2702557 A1 | 27-07-1978 |
| | | | FR | 2378282 A1 | 18-08-1978 |
| | | | GB | 1574681 A | 10-09-1980 |
| | | | JP | 53093684 A | 16-08-1978 |
| | | | SE | 7800725 A | 23-07-1978 |
| | | | US | 4187462 A | 05-02-1980 |
| WO 9218837 | A1 | 29-10-1992 | AU | 1545592 A | 17-11-1992 |
| | | | GB | 2254698 A | 14-10-1992 |
| | | | WO | 9218837 A1 | 29-10-1992 |
| | | | ZA | 9202536 A | 25-11-1992 |
| GB 2248301 | A | 01-04-1992 | KEINE | | |
| DE 1573236 | A1 | 10-02-1972 | DE | 1573236 A1 | 10-02-1972 |
| | | | GB | 1108684 A | 03-04-1968 |
| | | | US | 3391576 A | 09-07-1968 |
| DE 10127978 | C1 | 14-11-2002 | KEINE | | |
| DE 3709665 | A1 | 06-10-1988 | KEINE | | |
| GB 2260407 | A | 14-04-1993 | KEINE | | |

EPO FORM P0461

Für nähere Einzelheiten zu diesem Anhang : siehe Amtsblatt des Europäischen Patentamts, Nr.12/82